# EUROPEAN PATENT SPECIFICATION

(11) **EP 3 166 534 B1**
(45) Date of publication and mention of the grant of the patent: **17.04.2019**
(21) Application number: 15738822.4
(22) Date of filing: 10.06.2015
(51) Int. Cl.: A61D 19/02, A61D 19/04, A61B 17/43

(54) **DEVICE FOR ENTERING THE UTERUS**
VORRICHTUNG ZUM VORDRINGEN IN DIE GEBÄRMUTTER
DISPOSITIF D'INTRODUCTION DANS L'UTÉRUS

(30) Priority: 13.06.2014 CZ 20140411
(43) Date of publication of application: 17.05.2017
(73) Proprietor: Labmediaservis S.r.o., 54401 Dvur Králové nad Labem (CZ)
(72) Inventor: KREJCI, Tomás, 54401 Dvur Králové nad Labem (CZ)
(74) Representative: Skoda, Milan
(86) International application number: PCT/CZ2015/000059
(87) International publication number: WO 2015/188795

(56) References cited:
- US-A- 5 536 243
- US-A1- 2012 310 040

## Description

### Technical Field

The invention concerns the device for entering the uterus, especially the uterus of animals, especially cows, where the device includes a tube, where is placed a device for sampling, especially sampling for further microbiological examination.

### State of the Art

There is presently known a wide range of devices purposed for entering the uterus of animals.

From the patent document CS 246855 there is known a device for inserting of early embryos consisting of three tightly joined tubes with the same inner diameter. The first tube is smooth, the second one has a stopper and a head, and the third one has an olive tip, and thru the whole device goes a stick.

From another patent document CS 249975 there is known a plastic device for obtaining early embryos from cattle. This device consists of cone- shaped embouchure, which is connected to a flexible tube at its narrowed tip, which is equipped with a sheath, which rounded at its inlet side, while thru the flexible tube goes a tough stick, which is terminated by the handle above the cone shaped embouchure.

In patent CZ 294441 there is described a device for insemination of farm animals, like for example a sow, which contains catheter designed for entering genital tract of animals and enables releasing of spermatozoa. This device also includes a small tank designed to be joined with the catheter.

From the utility model CZ 25594 U there is known a device for obtaining ova and the follicular fluids. The leading tube is made of a metal tube, and is placed tightly in the upper hole of the holder of the probe. The leading tube has a narrowed opening, thru which passes an aspirating needle, while the back part and the whole body of the leading tube is broad enough to enable inserting and manipulating the needle. It moves through the body of the leading tube together with the needle during aspiration. The body of the leading tube includes in its inner part a duct for intake of follicular fluid during aspiration, while the duct opens at the conical- shape narrowed tip at the front end of the leading tube, there is attached an aspiration needle, and at the back part the leading tube is equipped with an opening with part for attaching of the aspiration tube.

From the European patent EP 1414379 there is known a retrievable intrauterine device for insertion of elements encapsulated in said device into uterine cavity, while the elements are from the following group: embryo, ova and/ or spermatozoa, fertilized egg, egg- cell and combinations of the previous elements, where the device contains a selectively permeable membrane with pores of certain size, which enables nutrients to penetrate to the elements.

From the patent application US2012310040 there is known the artificial insemination device may include a condom having a sheath and a reinforced cup that caps one end of the sheath, thereby having an inner concave surface and an outer convex surface; and a delivery handle comprising an elongate extension sized, shaped, shaped to contact the outer convex surface of the condom cup.

From the other patent application US5536243 there is known the self-contained time-release artificial insemination device which introduces a bolus of semen into the cervical canal or uterus over a period of hours is disclosed. The device includes a cervical cap adapted to conform and adhere to the cervix and includes an elongated nipple that extends in a perpendicular direction from the cap for insertion into the cervical canal or uterus.

The disadvantage of the devices for entering the uterus above is the fact that they do not enable the safe and sterile taking of microbiological samples.

From the state of the art there are also know the devices for entering the uterus of animals purposed for sterile microbiological sampling, which are equipped with a closing device, which is partly perforated, while the perforation is not permeable. During sampling is the closing device perforated by the device for sampling and it is possible to perform a sterile sampling. The important disadvantage of this device is the fact that the closing device could be removed and stays in the uterus of an animal. It could cause infertility, because a piece of plastic acts as contraception. The next disadvantage is the fact that the closing device could be damaged before sampling itself, which could cause devaluation of the sample.

From the state of the art above it is evident that there it is not known any device for sterile sampling of uterus without risk of caused infertility of the animal.

The goal of the invention is a device for entering the uterus, which is simple, while it provides a microbiological sampling without limitation of the further usability of the farm animal.

### Principle of the Invention

The disadvantages of the devices and method mentioned above removes and the solution offers the device for entering the uterus, especially the uterus of animals, especially cows, and for sampling, especially for microbiological sampling, which comprises a tube, where is placed a device for sampling, especially for microbiological sampling, an inserting cap which is at the front end of the tube, wherein the inserting cap is nonpermeable, and is made of a biodegradable material, according to the invention whose essence is in the fact that the inserting cap is put onto an outer wall of the tube, is rounded to simplify entering the uterus, is not perforated, and can be taken off at the moment of sampling. The inserting cap usually is not perforated, and at the moment of sampling it is taken off and afterwards it spontaneously decomposes in the uterus. It guarantees a safe sterile sampling and it does not influence fertility of the animal at the same time.

To ensure 100% sterility it is suitable if the biodegradable material is not permeable.

In its advantageous version is the biodegradable material made of proteins, while the most advantageous biodegradable material is gelatine.

Furthermore, it is advantageous when the shape of the cap is adapted for entering the uterus, and the shape could be specific for each certain animal species.

Concerning the optimal utilization of the device it is advantageous when the cap is put onto an outer part of the tube.

The device for entering the uterus enables a safe and sterile microbiological sampling without harming the animal and without negative influencing of reproductive abilities of the animal due to the cap which decomposes in the uterus.

### Overview of the Figures

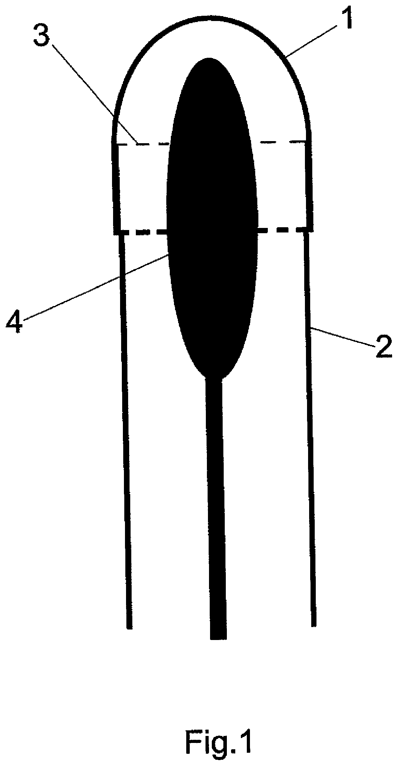

The invention is in detail described in the drawing, where in the Fig. 1 there is depicted a detailed view of the front end of the device for entering the uterus.

### Examples of the Performance of the Invention

The device for entering the uterus of the animal (Fig. 1), especially cows which contains a tube 2, where is placed a device 4 for microbiological sampling, which is used by determination of antibiotics susceptibility by endometritis in cows and for determination of trichomonasis in cows.

The tube 2 is made of plastic. The device for sampling 4 is the plastic stick with a swab.

The device can be alternatively used for insertion of elements, drugs or embryos into uterus.

At the front end 3 of the tube 2 there is put an inserting cap 1, which is made of a nonpermeabile biodegradable material, which is from proteins and is made of gelatine.

An inserting cap 1 is put onto an outer wall of the tube 2, while it is rounded to simplify entering the uterus.

### Industrial Application

The device for entering the uterus is purposed to be used for microbiological sampling of the uterine wall in animals, especially cows.

### List of Reference Marks

- 1: Inserting cap
- 2: Tube
- 3: Front end
- 4: Device for sampling

## Claims

1. A device for entering uterus, especially uterus of animals, especially cows, and for sampling, especially for microbiological sampling, which comprises:
a tube (2), where is placed a device (4) for sampling, especially for microbiological sampling,
an inserting cap (1) which is at the front end (3) of the tube (2), wherein the inserting cap (1) is nonpermeable, and is made of a biodegradable material, **characterised by that**
the inserting cap (1) is put onto an outer wall of the tube (2), is rounded to simplify entering the uterus, is not perforated, and can be taken off at the moment of sampling.

2. The device for entering the uterus of one of the claim 1 **characterised by that** the biodegradable material is protein.

3. The device for entering the uterus of one of the previous claims **characterised by that** the biodegradable material is gelatine.

## Patentansprüche

1. Die Vorrichtung für den Eintritt in die Gebärmutter, insbesondere für den Eintritt in die Gebärmutter der Tiere, vor allem der Kühe, sowie für die Entnahme, vor allem als Mittel für die Entnahme eines mikrobiologischen Befundes, die enthält:
ein Röhrchen (2), in dem ein Mittel (4) für die Entnahme enthalten ist, insbesondere für die Entnahme eines mikrobiologischen Befundes,
einführende Kappe (1), die an der Stirn (3) des Röhrchens (2) angebracht ist,
wobei die einführende Kappe (1) nicht porös und aus dem biodegradabilen Material hergestellt ist, **ist dadurch gekennzeichnet, dass** die einführende Kappe (1) auf die Außenseite des Röhrchens (2) aufgezogen wird, abgerundet ist, um den Eintritt in die Gebärmutter zu erleichtern, nicht perforiert ist und zum Zeitpunkt der Entnahme herunter genommen werden kann.

2. Die Vorrichtung für den Eintritt in die Gebärmutter nach der Forderung 1, **ist dadurch gekennzeichnet, dass** das biodegradabile Material Eiweißstoff ist.

3. Die Vorrichtung für den Eintritt in die Gebärmutter nach einer der vorherigen Forderungen, **ist dadurch gekennzeichnet, dass** das biodegradabile Material Gelatine ist.

## Revendications

1. Le dispositif pour entrer dans le vagin, notamment le dispositif pour entrer dans le vagin des animaux, notamment des vaches, et pour le prélèvement, notamment le moyen pour le prélèvement de la matière microbiologique, qui contient :
une petite tube où il est placé le moyen (4) pour le prélèvement, notamment le moyen pour le prélèvement de la matière microbiologique,
le chapeau d'introduction (1) qui se trouve sur le front (3) de la petite tube (2) et en même temps le chapeau n'est pas poreux et il est fabriqué de la matière biodégradable,
**caractérisé par le fait, que**
le chapeau d'introduction (1) est mis sur la surface extérieure de la tube (2),
le chapeau est arrondi pour la simplification de l'entrée dans le vagin, il n'est pas perforé et il peut être retiré au moment du prélèvement.

2. Le dispositif pour entrer dans le vagin selon la revendication 1 **caractérisé par le fait, que** la matière biodégradable est la protéine.

3. Le dispositif pour entrer dans le vagin selon une des revendications précédentes, **caractérisé par le fait, que** la matière biodégradable est une gélatine.
